# EUROPÄISCHE PATENTSCHRIFT

(11) **EP 1 674 032 B1**
(45) Veröffentlichungstag und Bekanntmachung des Hinweises auf die Patenterteilung: **18.01.2012**
(21) Anmeldenummer: 05028141.9
(22) Anmeldetag: 22.12.2005
(51) Int. Cl.: A61B 1/00

(54) **Verfahren und Vorrichtung zum Applizieren von elastischen Schläuchen auf Endoskope**
Method and device for applying an elastic sheath to an endoscope
Procédé et dipositif pour placer un tuyau élastique sur un endoscope

(30) Priorität: 23.12.2004 DE 102004063400
(43) Veröffentlichungstag der Anmeldung: 28.06.2006
(73) Patentinhaber: Rank, Norbert, Dr., 86199 Augsburg (DE); Rueff, Karl, Dr., 86159 Augsburg (DE)
(72) Erfinder: Rank, Norbert, Dr., 86199 Augsburg (DE); Rueff, Karl, Dr., 86159 Augsburg (DE)
(74) Vertreter: Ernicke, Klaus Stefan

(56) Entgegenhaltungen:
- US-A- 4 646 722
- US-A- 4 907 395
- US-A- 4 991 564
- PATENT ABSTRACTS OF JAPAN Bd. 017, Nr. 221 (C-1054), 7. Mai 1993 (1993-05-07) & JP 04 357920 A (ASAHI OPTICAL CO LTD), 10. Dezember 1992 (1992-12-10)

## Beschreibung

Die Erfindung betrifft ein Verfahren und eine Vorrichtung zum Applizieren von elastischen Schläuchen auf Endoskope mit den Merkmalen im Oberbegriff des Verfahrens- und Vorrichtungshauptanspruchs.

Wenn in der medizinischen Praxis Endoskope ohne Umhüllung benutzt werden, müssen sie vor jeder Benutzung gereinigt und desinfiziert werden. Dies ist aufwändig und belastet das Endoskop.

Die US-4,991,564 A zeigt ein Verfahren und eine Applikationsvorrichtung zum Aufbringen elastischer Schläuche auf Endoskope. Der mit dem freien Ende auf einer im wesentlichen durchmessergleichen, versteifenden Hülse befestigte Schlauch wird mittels einer Druckluftquelle über eine separate Halterung und eine Durchgangsbohrung beaufschlagt. Der Schlauch ist am distalen Ende mit einem Deckel verschlossen und wird vor dem Einführen des Endoskops vollständig aufgeblasen und axial sowie radial gedehnt. Im Schlauch ist ein zweiter separater Schlauch verlegt, der mit einer umfangsseitigen Nut des Endoskops korrespondiert und der die Durchführung einer Zange und eine Probenentnahme erlaubt. Für die Drucklufteinspeisung ist eine Manschette mit einer Ringbohrung oder einem Druckluftanschluss vorhanden, die über ein mit dem hinteren Ende des Schlauchs verbundenes massives Mundstück geschoben werden kann und mit einer dortigen Mantelbohrung zusammenwirkt. Das Mundstück hat am rückwärtigen Ende eine Dichtung für den Schlauchabschluss am Endoskop.

Aus der JP 04-357920 A ist eine Schlauchhalterung bekannt, die einen Druckluftanschluss aufweist und frontseitig einen Stutzen zum Aufnehmen eines etwas geweiteten Schlauchendes hat. Der Schlauch hat auch hier am Vorderende einen Verschlussdeckel und wird vor dem Einführen des Endoskops vollständig aufgeblasen. Das Endoskop besitzt ein biegeelastisches Vorderende. Der Schlauchhalter hat einen am vorerwähnten Stutzen mündenden Durchgangskanal, an dessen rückwärtigem Ende eine Dichtung angeordnet ist.

Die US 4,646,722 A befasst sich mit der Applikation eines elastischen Schlauches auf einem Endoskop, wobei in der einen Ausführungsform der Schlauch ausgerollt und dann auf dem durchgesteckten Endoskop wieder abgerollt wird. In einer Variante ist am rückwärtigen Schlauchende ein Aufblaskragen angeordnet, der einen zylindrischen Körper und eine Aufblasdüse sowie eine Ringdichtung und einen Sitz aufweisen. Auf diesem Sitz ist außenseitig das rückwärtige Ende des elastischen Schlauches befestigt. Der Aufblaskragen und der elastische Schlauch sind fest miteinander verbunden. Am anderen Schlauchende ist eine zylindrische Endkappe mit einem durchsichtigen Fenster angeordnet. Auch hier wird zunächst der gesamte Schlauch aufgeblasen und geweitet, bevor wird das Endoskop eingeführt wird.

Die US 4,907,395 A zeigt eine ähnliche Applikationsvorrichtung wie der vorgenannte Stand der Technik.

Es ist Aufgabe der vorliegenden Erfindung, die Handhabbarkeit von Endoskopen zu verbessern und hierfür eine geeignete Technik bereitzustellen.

Die Erfindung löst diese Aufgabe mit den Merkmalen im Verfahrens- und Vorrichtungshauptanspruch. Die Erfindung sieht vor, dass zur Verbesserung der Hygiene ein dünnwandiger elastischer Schlauch auf das Endoskop appliziert wird. Dies geschieht mittels eines erfindungsgemäßen Applikationsverfahren und einer zugehörigen Applikationsvorrichtung.

Die beanspruchte Applikationstechnik hat den Vorteil, dass der Schlauch einfach und ohne größere Beschädigungsgefahr auf das Endoskop aufgezogen werden kann. Mit dieser Technik lassen sich auch andere dünnwandige und dehnelastische Schläuche auf andere längliche Gegenstände aufziehen. Die Durchmesser der Endoskope bzw. länglichen Gegenstände können variieren, wobei unterschiedliche Aufziehhülsen und ggf. auch austauschbare Dichtungen eingesetzt werden. Die Applikationsvorrichtung braucht ansonsten nicht geändert zu werden.

Die Applikationsvorrichtung erfordert einen geringen Bauaufwand. Sie besteht aus wenigen Teilen und lässt sich kostengünstig herstellen. Sie kann mit jeder Art von Fluiden, vorzugsweise mit Druckluft, betrieben werden. Die Applikationsvorrichtung lässt sich leicht und einfach handhaben. Bei Einsatz einer externen Fluidquelle kann sie auch sehr klein bauen.

Über den elastischen Schlauch kann außerdem zumindest temporär ein weiterer Schlauch oder eine Hülle aufgezogen werden, die weniger oder nicht dehnbar sind. Diese Zusatzmaßnahme verhindert, dass der elastische Schlauch sich unter Einwirkung des Druckfluids zu weit seitlich dehnt, sondern sich eher nach distal dehnt und damit den Vorschub des Endoskops in den Schlauch erleichtert. Der zweite Schlauch bzw. die Hülle können anschließend können anschließend wieder entfernt werden.

In den Unteransprüchen sind weitere vorteilhafte Ausgestaltungen der Erfindung angegeben.

Die Erfindung ist in den Zeichnungen beispielsweise und schematisch dargestellt. Im Einzelnen zeigen:
- Figur 1:: Eine Applikationseinrichtung mit einer Aufziehhülse, einem Schlauch und einem Endoskop im Längsschnitt und in der Ausgangsposition,
- Figur 2:: die Applikationsvorrichtung von Figur 1 in Betriebsstellung mit einem teilweise in den Schlauch eingeführten Endoskop,
- Figur 3:: eine Stirnansicht der Applikationsvorrichtung von Figur 1 mit Blick auf die Auslassseite,
- Figur 4:: eine Stirnansicht der Applikationsvorrichtung von Figur 1 mit Blick auf die Einlassseite
- Figur 5:: eine Stirnansicht der Applikationsvorrichtung gemäß Figur 3 in geöffneter Stellung,
- Figur 6:: eine zweiteilige Variante der Aufziehhülse mit integrierter Dichtung,
- Figur 7:: eine dreiteilige Variante der Aufziehhülse mit Schlauch,
- Figur 8:: eine Variante der Applikationseinrichtung für die Aufziehhülsen von Figur 6 und 7 in vereinfachter Darstellung,
- Figur 9 und 10:: eine weitere Variante der Applikationseinrichtung in Draufsicht und Seitenansicht und
- Figur 11:: eine Variante des Schlauchs.

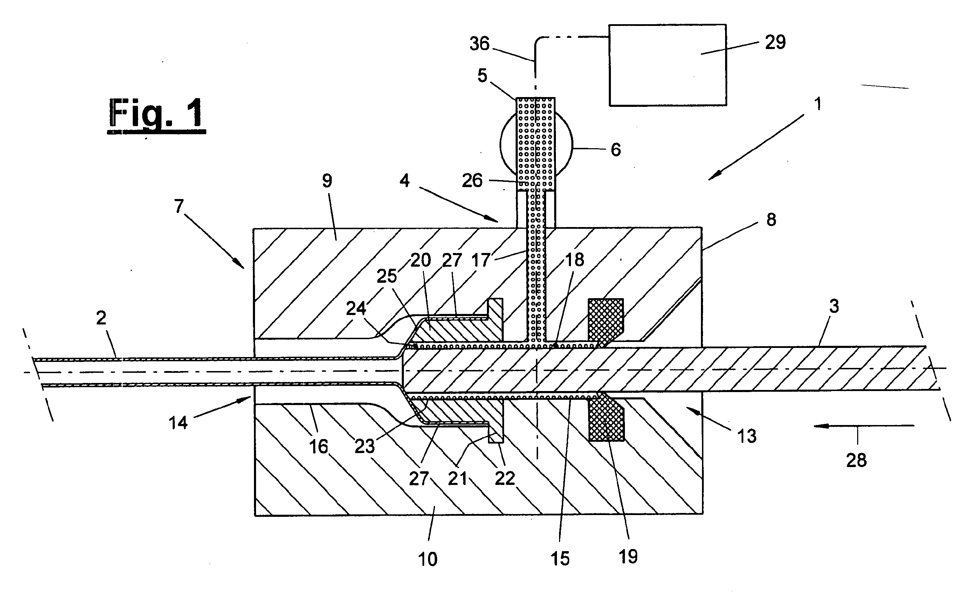

Figur 1, 8 und 10 zeigen im Längsschnitt bzw. eine Applikationsvorrichtung (1) zum Applizieren von dünnwandigen elastischen Schläuchen (2) auf Endoskope (3) oder auf andere längliche Gegenstände. Das Endoskop (3) ist in den Zeichnungen vereinfacht dargestellt. Es besteht aus einem zylinderrohrförmigen Schaft mit einer Optik am vorderen Stirnende. Über diesen formstabilen Schaft wird der Schlauch (2) aufgezogen und dicht angelegt.

Der Schlauch (2) besteht aus einem körperverträglichen und vorzugsweise transparenten Material, z.B. aus Silicon oder einem anderen geeigneten Kunststoff. Der längliche Schlauch (2) kann an beiden Enden offen sein. Alternativ kann er an einem Ende temporär geschlossen sein, z.B. durch einen nach dem Aufziehen wieder geöffneten Knoten, eine Klemme oder dgl.. Der Schlauch (2) kann an einem Ende auch dauerhaft geschlossen und z.B. sackartig ausgebildet sein. Die beidseitig offene und ggf. durch einen Knoten nur temporär geschlossene Schlauchform hat Vorteile für die am distalen Endoskopende häufig angebrachten Optik-Komponenten, Schneid- und Greifwerkzeuge etc..

Die Elastizität des Schlauchs (2) ist zumindest in Umfangsrichtung gegeben, so dass der Schlauchdurchmesser geweitet werden kann. Zusätzlich kann eine Dehnelastizität in Längsrichtung vorhanden sein. Das Endoskop (3) wird in der mit Pfeil angegebenen Einführrichtung (28) in den Schlauch (2) eingeführt.

Zum Einführen des Endoskops (3) wird der Schlauch (2) mit einem unter Druck eingefüllten Fluid (26) geweitet, wobei der Fluidraum (18) abgedichtet wird. In diesen geweiteten Schlauchbereich wird das Endoskop (3) eingeführt, wobei die Schlauchaufweitung mit dem Vorschub des Endoskops Schritt hält und zwischen Schlauch und Endoskop eine Fluidschicht verbleibt. Zu Beginn des Aufziehprozesses kann der Schlauch (2) mit seinem offenen Endbereich (27) auf eine weitende Aufziehhülse (20) aufgezogen und dort durch Reibung, Klemmung oder auf andere geeignete Weise festgehalten werden. Alternativ können vorgefertigte und bereits mit einer Aufziehhülse (20) versehene Schläuche (2) eingesetzt werden.

Durch den innenliegenden Hülsenkanal (23) wird das Endoskop (3) mit umfangsseitigen Spiel eingeführt. Zwischen dem Endoskop (3) und dem im Durchmesser größeren Hülsenkanal (23) besteht ein Freiraum (24), in den das extern und im rückwärtigen Bereich zugeführte Fluid (26) einströmen und zur Hülsenvorderseite (25) gelangen kann. Der Freiraum (24) bildet einen Teil des vorerwähnten Fluidraums (18). Der dünnwandige und dehnelastische Schlauch (2), der z.B. eine Wandstärke von 0,5 mm und kleiner hat, verschließt mit seiner starken trichterartigen Aufweitung die vordere Düsenkanalöffnung und liegt auch am Endoskopende an. Das Fluid kann somit nicht ungehindert durch das freie distale Schlauchende ausströmen. Unter Einwirkung des druckbeaufschlagten Fluids dehnt und weitet sich der Schlauch in diesem Bereich und hebt sich radial vom Endoskopende ab. Durch die entstehende Durchmesservergrößerung kann das Endoskop (3) ohne Zwängung langsam in den Schlauch (2) eingeführt und weiter bis zum distalen Schlauchende geschoben werden, wobei die Schlauchdehnung mitwandert. Nach Abschalten der Fluidversorgung schrumpft der dehnelastische Schlauch (2) und sitzt fest und dicht auf dem Mantel des Endoskops (3).

Die Applikationsvorrichtung (1) kann als komplettes Set aus einem Halter (7) mit einem axialen Durchführkanal (15), mindestens einer Aufziehhülse (20), mindestens einer Dichtung (19) und einer Fluidzuführung (4) bestehen.

Das Fluid (26) kann von beliebiger Art sein. Im gezeigten Ausführungsbeispiel handelt es sich um Druckluft. Die Fluidzuführung (4) bzw. die Applikationsvorrichtung (1) können eine externe Fluidquelle (29), z.B. eine Druckluftquelle, aufweisen, die über eine geeignete Fluidleitung (36) mit einem Fluidanschluss (5), insbesondere einem Druckluftanschluss, am Halter (7) verbunden ist. Alternativ kann in den Halter eine kleine Luftpumpe integriert sein, die Außenluft ansaugt. Die Fluidzuführung (4) ist in diesem Fall mit einer geeigneten Energieversorgung und Steuerung versehen.

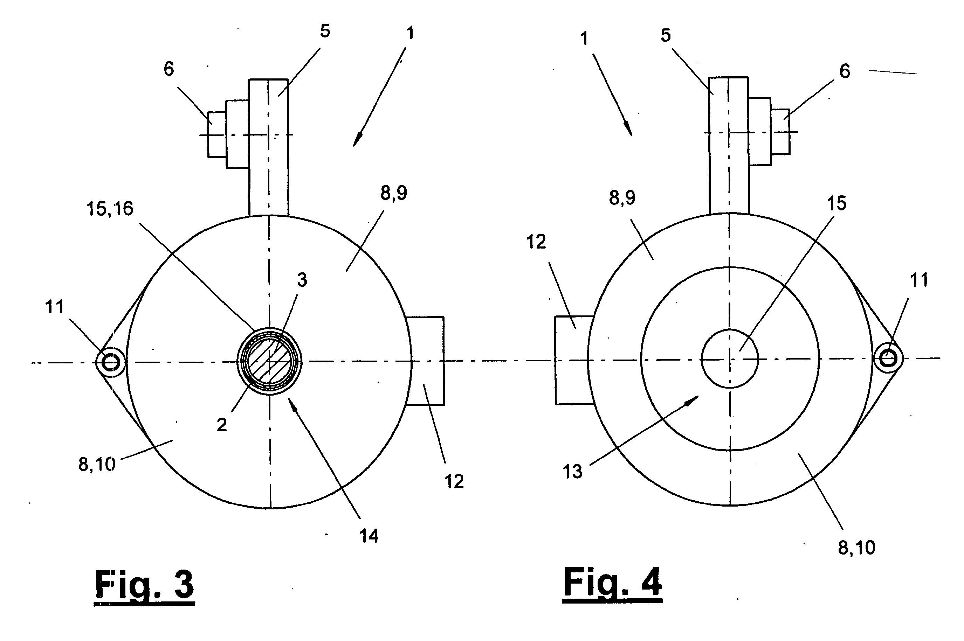

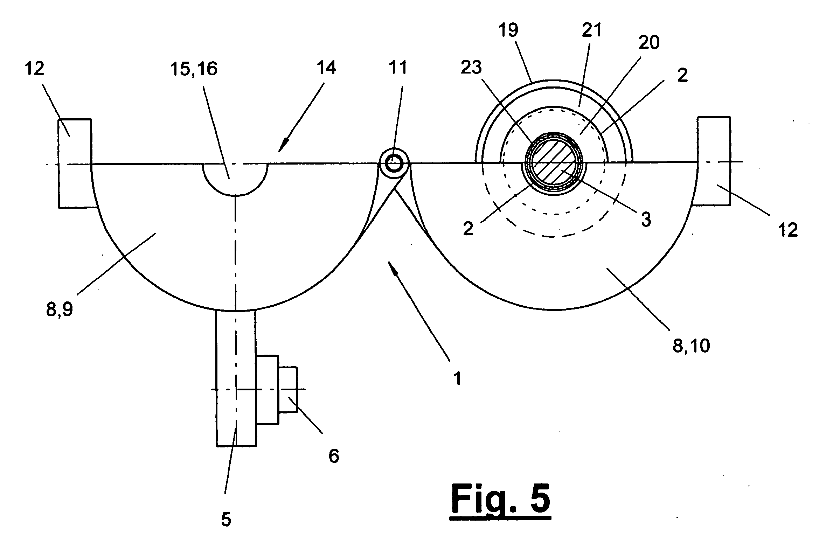

Der Halter (7) kann in der Ausführungsform von Figur 1 bis 5 geöffnet werden und besitzt hierzu ein Gehäuse (8) aus mehreren lösbaren Teilen (9,10). In der gezeigten und bevorzugten Ausführungsform ist das Gehäuse (8) in der Länge mittig geteilt und besteht aus zwei Gehäuseteilen (9,10), die über ein Gelenk (11) drehbar miteinander verbunden sind. Das Gelenk (11) kann eine permanente Drehverbindung oder eine Steckverbindung sein. Statt dessen kann auch jede andere geeignete Verbindung (17) vorhanden sein. Auf der anderen Seite der Gehäuseteile (9,10) ist ein entsprechender Verschluss (12) vorhanden, mit dem in Schließstellung die Gehäuseteile (9,10) fest miteinander verbunden werden können.

Der Durchführkanal (15) ist im Inneren des Gehäuses (8) und vorzugsweise an der Trennstelle der Gehäuseteile (9,10) angeordnet, so dass beide Teile (9,10) je eine Kanalhälfte aufweisen. Der Durchführkanal (15) besitzt an seinen Stirnseiten eine Einlassöffnung (13) und eine Auslassöffnung (14). Im Gehäuseinneren ist zwischen den Öffnungen (13,14) ein Aufnahmebereich (16) für die Aufziehhülse (20) und für den Schlauch (2) vorgesehen.

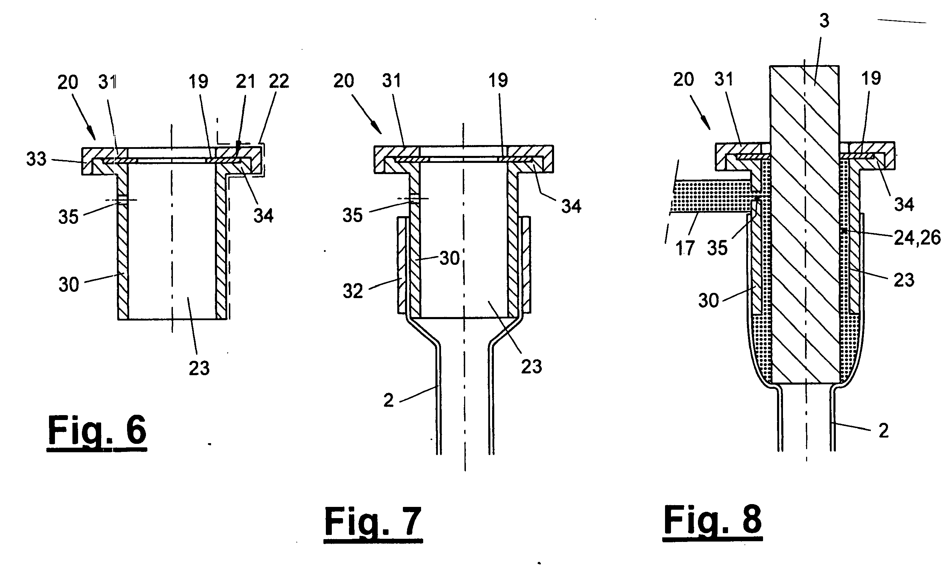

Die Aufziehhülse (20) ist im Ausführungsbeispiel von Figur 1 bis 5 einteilig ausgebildet. Figur 6 bis 7 zeigen mehrteilige Varianten.

Die Aufziehhülse (20) besitzt in allen Varianten einen inneren Hülsenkanal (23), der an die Querschnittsform des Endoskops (3) angepasst ist und der einen geringfügig größeren Durchmesser als das Endoskop (3) aufweist. Hierdurch wird ein umfangseitiger Freiraum (24) gebildet. An geeigneter Stelle, vorzugsweise an der Rückseite, besitzt die Aufziehhülse (20) einen radial vorstehenden Bund (21), der mit einer entsprechenden formschlüssigen Hülsenführung (22) in den Gehäuseteilen (9,10) zusammenwirkt. Alternativ kann die formschlüssige Führung auch auf andere Weise, z.B. mit einem Bund an den Gehäuseteilen (9,10) und einer Aufnahmenut an der Aufziehhülse (20) ausgebildet sein. Ferner kann die Hülse auch auf andere Weise, z.B. durch Kraftschluss mit Klemmung in den Gehäuseteilen (9,10) befestigt werden. Die lose Aufziehhülse (20) lässt sich bei geöffnetem Gehäuse (8) entnehmen und austauschen. Für unterschiedliche Endoskopdurchmesser können unterschiedliche Aufziehhülsen (20) vorhanden sein, die entsprechend differierende Innendurchmesser des Hülsenkanals (23) aufweisen. Auch der Außendurchmesser des zylindrischen Schlauchaufziehbereichs kann variieren. Um das anfängliche Aufziehen des Schlauches zu erleichtern, kann die Aufziehhülse (20) eine zum distalen Ende sich verjüngende, z.B. konisch angeschrägte oder angefaste Vorderseite (25) und weiche Rundungen an den Wandübergängen aufweisen.

Der Fluidanschluss (5) ist in der vorerwähnten Weise außenseitig am Gehäuse (8) angeordnet und befindet sich z.B. an der oberen Gehäusehälfte (9). Der Fluidanschluss (5) besitzt ein von Hand steuerbares Ventil (6), mit dem die Fluidzufuhr geöffnet und geschlossen werden kann. Das Ventil (6) kann auch fernsteuerbar sein und einen Ventilantrieb aufweisen, wobei es z.B. als Magnetventil ausgebildet ist. Vom Fluidanschluss (5) führt ein kanalartiger Fluidzufluss (17) zum Durchführkanal (15) und mündet an dessen Umfang. Der Fluidzufluss (17) ist in Einführrichtung (28) mit Abstand vor der Aufziehhülse (20) angeordnet. Eventuell ist ein weiteres Ventil (nicht dargestellt) zur Druckregulierung und zum Ablassen von Überdruck vorhanden.

Im Ausführungsbeispiel von Figur 1 bis 5 ist am Durchführkanal (15) eingangsseitig mit Abstand vor dem Fluidzufluss (17) eine Dichtung (19) für das eingeführte Endoskop (3) angeordnet. Dies kann z.B. eine austauschbar im Gehäuse (8) gehaltene Ringdichtung sein, die eine innen liegende, ringförmige und flexible Dichtlippe aufweist, die sich eng und dichtend an den Außenumfang des Endoskops (3) anschließt. Die Dichtung (19) ist in Einführrichtung (28) mit Abstand hinter der trichterförmig sich verengenden Einlassöffnung (13) angeordnet, welche für eine Seitenführung beim Einführen des Endoskops (3) sorgt.

Der Durchführkanal (15) besitzt zumindest im Bereich der Dichtung (19) und der Aufziehhülse (20) einen größeren Durchmesser als das Endoskop (3), wodurch ein umfangseitiger Fluidraum (18) gebildet wird, der über den Zufluss (17) mit dem Fluidanschluss (5) in Verbindung steht. Der Fluidraum (18) setzt sich weiter fort in den Freiraum (24) in der Aufziehhülse (20). Rückseitig ist der Fluidraum bzw. die vom vergrößerten Durchführkanal (15) gebildete Fluidkammer durch die Dichtung (19) abgeschlossen. An der Vorderseite ist der Fluidraum (18) bzw. der Freiraum (24) durch die im Dehnbereich und an der Vorderseite (25) anliegende Schlauchwandung abgeschlossen. Der Durchführkanal (15) kann im Bereich der Fluidkammer (18) in seiner Formgebung an die Form des Endoskops (3) oder eines anderen länglichen Gegenstands angepasst sein. Im gezeigten Ausführungsbeispiel ist der Durchführkanal (15) zylindrisch. Im Aufnahmebereich (16) hat der Durchführkanal (15) eine trichterförmig aufgeweitete Form. Im Hülsenbereich ist der Durchmesser des zylindrischen Kanals soweit vergrößert, dass die Aufziehhülse (20) mit dem aufgezogenen Schlauchende (27) eingelegt werden kann. Hierbei können die Durchmesser derart aufeinander abgestimmt sein, dass sich eine Klemmführung ergibt und das Schlauchende (27) im Aufnahmebereich zwischen der Kanalwandung und der Aufziehhülse (20) eingeklemmt ist. Ansonsten hält es durch Reibschluss auf der Aufziehhülse (20). Der Aufnahmebereich (16) setzt sich am rückwärtigen Ende in die Ringnut der Hülsenführung (22) fort. Das in Einführrichtung (28) vordere Ende des Aufnahmebereichs (16) ist ebenfalls gegenüber dem Kanaldurchmesser im Bereich der Fluidkammer (18) erweitert und erlaubt das Durchführen des Endoskops (3) mit dem umfangsseitig aufgezogenen Schlauch (2). Gegenüber dem vorerwähnten Hülsenbereich ist dieser Kanalbereich allerdings verengt, um übermäßige Aufblähungen des Schlauches (2) zu verhindern. Das in Einführrichtung (28) vordere Ende des Aufnahmebereichs (16) bildet die eingangs erwähnte Auslassöffnung (14).

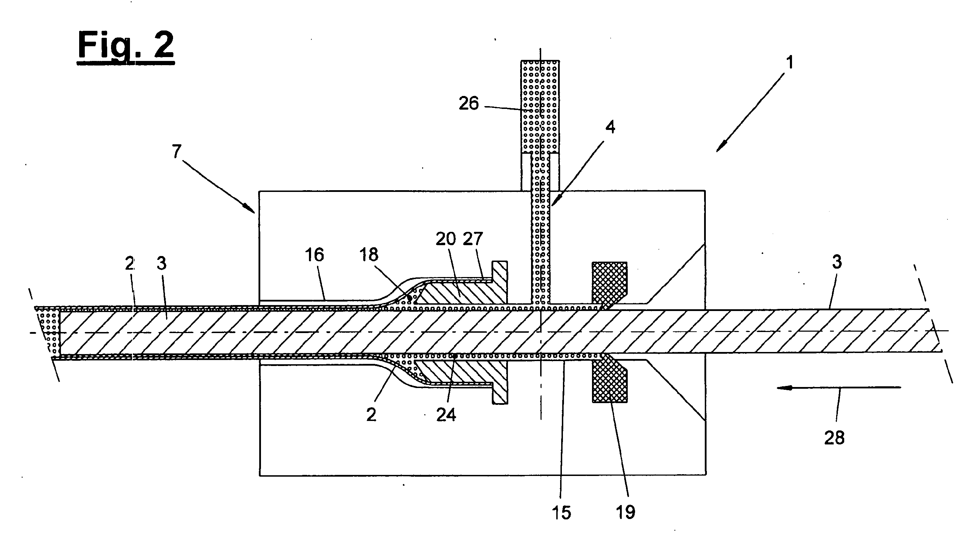

Figur 1 und 2 zeigen verschiedene Betriebszustände der Applikationsvorrichtung (1). In der Ausgangsstellung gemäß Figur 1 wird zunächst die Aufziehhülse (20) mit dem aufgezogenen Schlauchende (27) in das geöffnete Gehäuse (8) eingeführt und in den Aufnahmebereich (16) sowie die Hülsenführung (22) an einem der beiden Gehäuseteile (9,10) gelegt. Anschließend wird das Gehäuse (8) geschlossen und verriegelt. Innenseitige Gehäusedichtungen (nicht dargestellt) dichten dabei zusätzlich die Gehäuseteile (9,10) im fluidführenden Bereich gegeneinander ab, um Fluidleckagen zu vermeiden. Anschließend wird das Endoskop (3) durch die Einlassöffnung (13) und die Dichtung (19) sowie die Aufziehhülse (20) eingeführt und bis zu deren Vorderseite (25) geschoben.

Der Schlauch (2) hat im ungedehnten Ausgangszustand einen wesentlich kleineren Durchmesser als die Aufziehhülse (20). Die trichterartige, starke Schlauchaufweitung liegt an der Hülsenvorderseite (25) dicht an und erstreckt sich vom Hülsenmantel radial bis weit in den Hülsenkanal (23) hinein. Das Endoskop (3) wird bis in die Nähe oder bis zur Anlage an der Schlauchaufweitung vorgeschoben. Figur 1 zeigt diese Anordnung.

Anschließend wird die Fluidzufuhr geöffnet, wobei das unter Druck eingespeiste Fluid (26) durch die Fluidkammer (18) und den zwischen Hülsenkanal (23) und Endoskop (3) gebildeten ringkanalartigen Freiraum (24) bis zur Hülsenvorderseite (25) und der dort dicht anliegende Schlauchwandung fließt. Die Freiraum- oder Kanalöffnung wird durch die Schlauchwandung verschlossen. Das unter Druck stehende Fluid dehnt die Schlauchwandung und hebt sie etwas vom Endoskopende ab. Das Endoskop (3) kann mit seinem Vorderende in diesen Dehnungsbereich langsam vorgeschoben werden, wobei das Fluid (26) den Schlauch (2) weiterhin dehnt und auch umfangsseitig vom Endoskop (3) abhebt. Dank dieser Schlauchdehnung und des Fluidpolsters kann das Endoskop (3) bis zum anderen Schlauchende (nicht dargestellt) durchgeschoben werden. Nach Abschalten der Fluidzufuhr kann das Endoskop (3) mit dem aufgezogenen Schlauch (2) aus dem geöffneten Gehäuse (8) entnommen werden. Die Aufziehhülse (20) kann hierbei ggf. am hinteren Schlauch- und Endoskopende verbleiben. Sie kann alternativ auch nach hinten abgezogen werden. Der dehnelastische Schlauch (2) verdrängt das drucklose Fluid (26) und schmiegt sich eng an das Endoskop (3).

Ein Verbleiben der Abziehhülse (20) am Endoskop (3) hat den Vorteil, dass das Endoskop (3) unter Einsatz der Applikationsvorrichtung (1) wieder aus dem Schlauch (2) herausgezogen werden kann. Hierfür werden die Teile in der vorerwähnten Weise in das geöffnete Gehäuse (8) eingelegt, wobei nach Gehäuseschluss Fluiddruck angelegt wird, der den Schlauch (2) weitet und ein Herausziehen des Endoskops (3) bei festgehaltenem vorderen Schlauchende ermöglicht.

Figur 6 bis. 8 zeigen Abwandlungen der Applikationsvorrichtung (1) und der Aufziehhülse (20). Die Aufziehhülse (20) kann mehrteilig sein und eine integrierte Dichtung (19) aufweisen. Ferner kann ein Fluideinlass (35) am Mantel der Aufziehhülse (20) angeordnet sein. Im Ausführungsbeispiel von Figur 6 und 8 besteht die Aufziehhülse (20) aus zwei miteinander verbindbaren Hülsenteilen (30,31), zwischen denen eine ringförmige Dichtung (19) angeordnet und eingespannt ist. Das eine Hülsenteil (30) ist als zylindrisches Rohr mit einem endseitigen Kragen oder Flansch (34) ausgebildet. Im Mantel dieses Hülsenteils (30) kann im rückwärtigen Bereich und in der Nähe des Kragens (34) ein Fluideinlass (35) vorhanden sein, der z.B. aus ein oder mehreren Durchgangsbohrungen besteht, durch die von außen her ein Fluid (26) in den innenseitigen Hülsenkanal (23) eingefüllt werden kann. Auf dem Rohrteil des Hülsenteils (30) wird der Schlauch (2) aufgezogen.

Das zweite Hülsenteil (31) hat die Form einer zylindrischen Topfscheibe mit einem im Innenbereich ausgenommenen Scheibenkörper und einen am Scheibenkörperrand angebrachten axial gerichteten Bund (33). Der Bund (33) umgreift den Kragen (34) des ersten Hülsenteils (30). Die Dichtung (19) ist in eine Aussparung zwischen den Scheibenkörper und den Kragen (34) eingelegt und dabei beidseits zwischen den Hülsenteilen (30,31) formschlüssig geführt und eingespannt. Die Dichtung (19) ragt in den Hülsenkanal (23) und liegt am eingeführten Endoskop (3) dichtend an.

Das Hülsenteil (31) und der Kragen (34) bilden in der Eingriffstellung miteinander ein Hülsenbund (21) wie im ersten beschriebenen Ausführungsbeispiel, welcher in einer Hülsenführung (22) des Gehäuses (8) in analoger Weise geführt sein kann. Hierdurch wird auch der Zusammenhalt der Hülsenteile (30,31) und der Dichtung (19) formschlüssig gesichert. Die mehrteilige Aufziehhülse (20) kann in entsprechender Weise in einem Gehäuse (8) der Applikationsvorrichtung (1) untergebracht werden.

Figur 7 zeigt eine weitere Ergänzung in Form eines dritten Hülsenteils (32), welches eine ringförmige Spannhülse bildet, die außenseitig auf den am Hülsenteil (30) aufgezogenen Schlauch (20) geschoben wird. Der Schlauch (20) wird hierbei zwischen den Hülsenteilen (30,32) eingespannt und besser festgehalten. Ein solches Hülsenteil (32) kann auch im ersten Ausführungsbeispiel der Figuren 1 bis 5 Verwendung finden.

Gemäß Figur 8 wird bei der Aufziehhülse (20) mit der integrierten Dichtung (19) das Endoskop (3) durch die Dichtung (19) in den hier ebenfalls erweiterten Hülsenkanal (23) geschoben und zunächst bis zum vorderen Hülsenende bewegt. In den hierdurch gebildeten umfangsseitgen Freiraum (24) kann ein Fluid (26) unter Druck durch den Fluideinlass (35) eingespeist werden. Dies kann in unterschiedlicher Weise geschehen. Z.B. kann der Fluidzufluss (17) in Form eines Rüssels direkt am Fluideinlass (35) außenseitig angesetzt werden. Der aufgezogene Schlauch (2) endet vor dem Fluideinlass (35). Alternativ kann wie im erstgenannten Ausführungsbeispiel das Gehäuse (8) einen Aufnahmebereich (16) zum Einsetzen der Aufziehhülse (20) mit dem angebrachten Schlauch (2) aufweisen. Hierbei ist der Aufnahmebereich stirnseitig abgedichtet, so dass ähnlich wie im ersten Ausführungsbeispiel eine Fluidkammer gebildet wird, die sich im Fall der Ausführungsform von Figur 8 außenseitig um die Aufziehhülse (20) erstreckt und mit dem Fluideinlass (35) in Verbindung steht. Diese Fluidkammer ist dann entsprechend gegen Fluidverluste im Gehäuse abgedichtet.

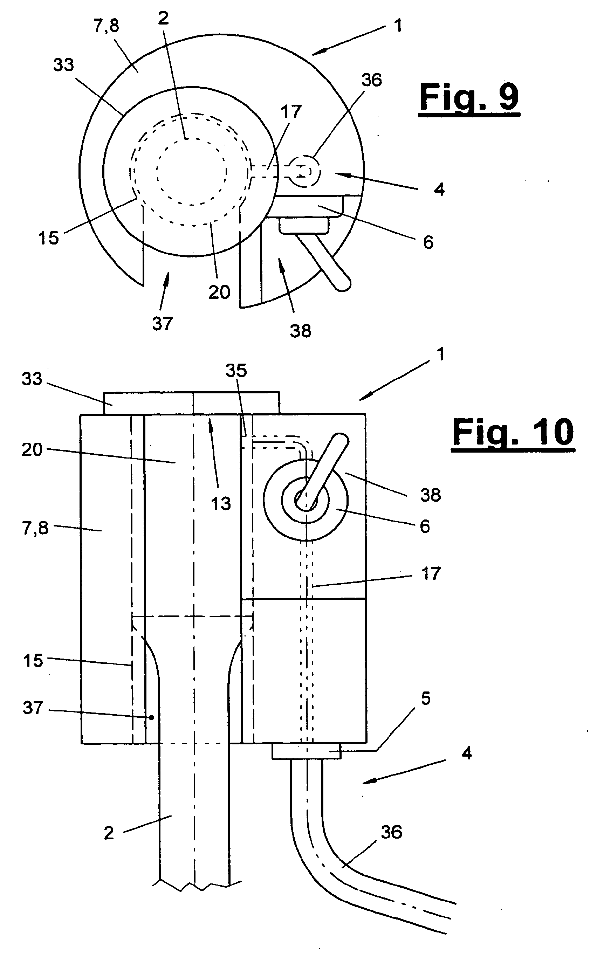

Figur 9 und 10 zeigen einen Halter (7) gemäß der vorliegenden Erfindung, der insbesondere für Aufziehhülsen gemäß Figur 6 bis 8 oder andere Ausführungsformen mit einem mantelseitigen Fluideinlass (35) geeignet ist. Der Halter (7) besitzt ein einteiliges, z.B. zylindrisches Gehäuse mit einem axialen Durchführkanal (15). Der Durchführkanal (15) kann außermittig angeordnet sein und eine seitliche Zugangsöffnung haben, die sich z.B. in der gezeigten Weise als Eintrittsschlitz (37) über die gesamte Kanallänge axial erstreckt. Die Schlitzweite ist kleiner als der Durchmesser des zylindrischen Durchlasskanals (15), so dass zwar der Schlauch (2) durch den Schlitz (37) seitlich eingeführt werden kann, wobei die Aufziehhülse (20) angehoben ist und anschließend in die Einlassöffnung (13) des Durchführkanals (15) eingeführt wird. Die im Durchmesser an den Durchlasskanal (15) angepasste Aufziehhülse (20) ist mit dem aufgezogenen Schlauch (2) dann formschlüssig im Durchführkanal (15) geführt und gehalten, wobei sie sich mit dem verbreiterten Bund (33) an der Gehäuseaußenwand abstützt.

Im Halter (7) bzw. im Gehäuse (8) ist in der Wandung ein Fluidzufluss-Kanal verlegt, der außenseitig an einem Fluidanschluss (5) mündet und mit der Fluidleitung (36) verbunden werden kann. Innenseitig mündet der Fluidzufluss (17) an der Wandung des Durchführkanals(15), wobei der im Hülsenmantel entsprechend angeordnete Fluideinlass (35) genau gegenüber liegt. Das Fluid kann durch diese Anordnung ohne signifikante Druckverluste in den Hülsenkanal (23) strömen. Über ein Ventil (6), das z.B. in einer Gehäuseaussparung (38) angeordnet ist, kann die Fluidzufuhr gesteuert bzw. geschaltet werden. Die Dichtung (19) ist bei dieser Ausführungsform an der Aufziehhülse (20) angeordnet.

Der in Figur 9 und 10 gezeigte Halter (7) hat eine besonders kompakte Bauform und ein kleines Gewicht. Er lässt sich mit einer Hand greifen und bedienen, wobei mit der anderen Hand das Endoskop eingeführt wird.

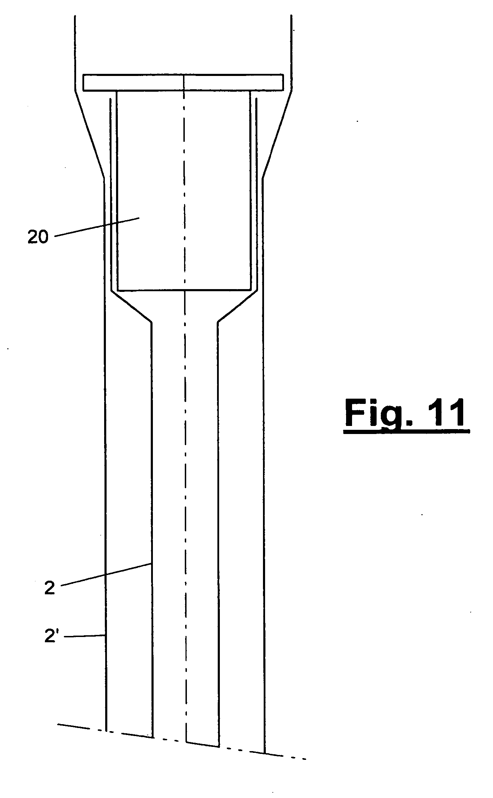

In Figur 11 ist eine Variante der Schlauchanordnung dargestellt. Über den elastischen Schlauch (2) wird hierbei ein weiterer Schlauch oder eine Hülle (2') aufgebracht, z.B. aufgezogen. Die weitere Hülle (2') kann an ein oder beiden Enden offen sein. Sie besitzt eine geringere Dehnelastizität als der aufzuweitende Schlauch (2). Die weitere Hülle (2') kann insbesondere eine nicht dehnbare Wandung aufweisen. Sie kann hierbei biegeelastisch oder auch starr sein. Der Innendurchmesser des zusätzlichen Schlauchs (2') ist größer als der Außendurchmesser des Endoskops mit dem aufgezogenen elastischen Schlauch (2). Die außenseitige zusätzliche Hülle (2') dient zur radialen Dehnungsbegrenzung des elastischen Schlauchs (2) unter Einwirkung des Fluiddrucks. Der Innendurchmesser der zusätzlichen Hülle (2') ist so groß gewählt, dass der elastische Schlauch (2) sich genügend weit radial dehnen kann, um einen ausreichenden Fluidraum (18) für das behinderungsfreie Einführen des Endoskops (3) zu bieten. Durch die umfangsseitige Begrenzung dehnt sich der elastische Schlauch (2) unter dem Fluiddruck nicht nur radial, sondern auch in ausreichender Weise axial und mit Richtung zum distalen Ende, so dass mit dem Endoskopvorschub die Schlauchaufweitung optimal fortschreitet.

Die zusätzliche Hülle (2') wird z.B. vor dem Aufziehen des elastischen Schlauchs (2) auf der Aufziehhülse (20) über dem Schlauch (2) angebracht. Dies kann alternativ auch nach dem Aufziehen auf der Hülse (20) geschehen. Am oberen Ende kann die zusätzliche Hülle (2') ebenfalls über die Aufziehhülse (20) gezogen sein und hat hierfür einen entsprechenden Innendurchmesser. Sie kann außerdem am oberen Ende über die Aufziehhülse (20) vorstehen.

In Abwandlung der gezeigten Ausführungsform kann die zusätzliche Hülle (2') als starrwandige Hülse ausgebildet sein, die axial längs des elastischen Schlauchs (2) beweglich ist und die mit dem Endoskopvorschub mitbewegt und dabei an der fortschreitenden Aufweitungsstelle des elastischen Schlauchs (2) gehalten wird. In der gezeigten Ausführungsform ist die zusätzliche Hülle (2') in geeigneter Weise festgelegt und z.B. an oder mit der Aufziehhülse (20) im Halter (7) eingespannt.

Abwandlungen der gezeigten Ausführungsform und der Verfahrenstechnik sind in verschiedener Weise möglich. Zum einen können statt eines Endoskops (3) beliebige andere längliche Gegenstände mit einem dehnelastischen Schlauch (2) überzogen werden. Die Gegenstandsformen können dabei beliebig variieren. Sie müssen nicht rotationssymmetrisch sein und müssen auch nicht über die Länge gleich bleiben. Der besagte Gegenstand (3) sollte allerdings eine ausreichende Formstabilität für die Einführbewegung aufweisen. Ansonsten kann die Applikationsvorrichtung (1) in konstruktiver Hinsicht variieren. Dies betrifft einerseits die Form und Lagerung der Aufziehhülse (20) sowie der Dichtung (19). Variabel ist auch die Gestaltung der Einlass- und Auslassöffnungen (13,14) sowie des Durchführkanals (15). Gleichermaßen kann das Gehäuse (8) in anderer Weise ausgebildet sein und aus mehr als zwei Teilen bestehen. Wenn das Endoskop (3) oder ein anderer länglicher Gegenstand in einem Zug durchgeführt werden können, ist auch eine komplette Öffnung des Gehäuses (8) nicht erforderlich. In diesem Fall genügt ein Öffnen des vorderen Gehäusebereichs zum Einführen und Entnehmen der Aufziehhülse (20) und des Schlauchs (2). Auch die Ausbildung des Schlauchs (2) ist beliebig wählbar. Der Schlauch (2) kann aus einem anderen dehnelastischen Material bestehen und muss nicht transparent sein. Zudem kann der Schlauch (2) beliebige Ausgangsdurchmesser aufweisen, die aus Gründen der Schrumpfhaltung nur kleiner als der Durchmesser des Endoskops (3) bzw. des länglichen Gegenstandes sein sollten. Ferner kann auch die Dichtung (19) austauschbar gestaltet sein und z.B. aus einem äußeren formstabilen Tragring bestehen, der in einer entsprechenden Führungsnut des Gehäuses (19) lösbar gelagert ist. Auf diese Weise lässt sich die Dichtung (19) ebenfalls austauschen, was im Verschleißfall oder zur Anpassung an unterschiedliche Durchmesser von Endoskopen (3) oder anderen länglichen Gegenständen erforderlich sein kann.

### BEZUGSZEICHENLISTE

- 1: Applikationsvorrichtung
- 2: Schlauch, Hülle
- 2': Schlauch, Hülle
- 3: Endoskop
- 4: Fluidzuführung
- 5: Fluidanschluss, Druckluftanschluss
- 6: Ventil
- 7: Halter
- 8: Gehäuse
- 9: Gehäuseteil
- 10: Gehäuseteil
- 11: Verbindung, Gelenk
- 12: Verschluss
- 13: Einlassöffnung
- 14: Auslassöffnung
- 15: Durchführkanal
- 16: Aufnahmebereich
- 17: Fluidzufluss
- 18: Fluidraum, Fluidkammer
- 19: Dichtung, Ringdichtung
- 20: Aufziehhülse
- 21: Bund
- 22: Hülsenführung
- 23: Hülsenkanal, Durchlassöffnung
- 24: Freiraum
- 25: Vorderseite
- 26: Fluid
- 27: Endbereich
- 28: Einführrichtung
- 29: Fluidquelle, Druckluftquelle
- 30: Hülsenteil
- 31: Hülsenteil
- 32: Hülsenteil
- 33: Bund
- 34: Kragen, Flansch
- 35: Fluideinlass
- 36: Fluidleitung
- 37: Schlitz
- 38: Gehäuseaussparung

## Patentansprüche

1. Verfahren zum Applizieren von dünnwandigen dehnelastischen Schläuchen (2) auf Endoskope (3), wobei der Schlauch (2) mit seinem offenen Endbereich (27) auf eine weitende Aufziehhülse (20) aufgezogen und dort festgehalten wird und die Aufziehhülse (20) mit dem aufgezogenen Schlauchende (27) in einem Halter (7) mit einer Fluidzuführung (6) angeordnet wird, wobei der Schlauch (2) mit einem eingefüllten Fluid (26) unter Druck geweitet wird und dass unter Abdichtung des Fluidraums (18) dabei das Endoskop (3) in den Schlauch (2) eingeführt wird, **dadurch gekennzeichnet, dass** die Aufziehhülse (20) mit dem aufgezogenen Schlauchende (27) in einen Halter (7) mit einem einteiligen Gehäuse (8) und mit einem seitlichen Einführschlitz (37) angeordnet wird.

2. Verfahren nach Anspruch 1, **dadurch gekennzeichnet, dass** durch einen innen liegenden Hülsenkanal (23) der Aufziehhülse (20) das Endoskop (3) mit umfangseitigem Spiel eingeführt wird.

3. Verfahren nach Anspruch 1 oder 2, **dadurch gekennzeichnet, dass** in einen umfangseitigen Freiraum (24) zwischen dem Endoskop (3) und dem Hülsenkanal (23) das Fluid (26) eingespeist wird.

4. Verfahren nach Anspruch 1, 2 oder 3, **dadurch gekennzeichnet, dass** der Schlauch (2) im ungedehnten Ausgangszustand einen wesentlich kleineren Durchmesser als die Aufziehhülse (20) aufweist, wobei die trichterartige, starke Schlauchaufweitung an der Hülsenvorderseite (25) dicht anliegt und wobei das Endoskop (3) bis in die Nähe oder bis zur Anlage an der Schlauchaufweitung vorgeschoben wird und die Schlauchaufweitung mit dem Endoskopvorschub fortschreitet.

5. Verfahren nach einem der vorhergehenden Ansprüche, **dadurch gekennzeichnet, dass** vor dem Einführen des Endoskops (3) über dem elastischen Schlauch (2) ein weniger dehnbarer weiterer Schlauch (2') oder eine Hülle aufgebracht wird.

6. Vorrichtung zum Applizieren von elastischen Schläuchen (2) auf Endoskope (3), wobei die Applikationsvorrichtung (1) einen Halter (7) mit einem Durchführkanal (15) und einer Fluidzuführung (4) sowie mindestens eine Aufziehhülse (20) und mindestens eine Dichtung (19) aufweist, **dadurch gekennzeichnet, dass** der Halter (7) ein einteiliges Gehäuse (8) mit einem seitlichen Einführschlitz (37) aufweist.

7. Vorrichtung nach Anspruch 6, **dadurch gekennzeichnet, dass** der Durchführkanal (15) außermittig angeordnet ist.

8. Vorrichtung nach Anspruch 6 oder 7, **dadurch gekennzeichnet, dass** der Eintrittsschlitz (37) sich über die gesamte Kanallänge des Durchlasskanals (15) axial erstreckt, wobei die Schlitzweite kleiner als der Durchmesser des zylindrischen Durchlasskanals (15) ist.

9. Vorrichtung nach Anspruch 6, 7 oder 8, **dadurch gekennzeichnet, dass** die Aufziehhülse (20) im Durchmesser an den Durchlasskanal (15) angepasst ist und mit dem aufgezogenen Schlauch (2) formschlüssig im Durchführkanal (15) geführt und gehalten ist, wobei sie sich mit einem verbreiterten Bund (33) an der Gehäuseaußenwand abstützt.

10. Vorrichtung nach einem der Ansprüche 6 bis 9, **dadurch gekennzeichnet, dass** die Applikationsvorrichtung (1) eine Fluidquelle (29) aufweist.

11. Vorrichtung nach einem der Ansprüche 6 bis 10, **dadurch gekennzeichnet, dass** der Durchführkanal (15) stirnseitig Einlass- und Auslassöffnungen (13,14) und dazwischen einen Aufnahmebereich (16) für die Aufziehhülse (20) und den Schlauch (2) aufweist.

12. Vorrichtung nach einem der Ansprüche 6 bis 11, **dadurch gekennzeichnet, dass** die Aufziehhülse (20) einen inneren Hülsenkanal (23) für das Endoskop (3) und außenseitig einen Bund (33) aufweist.

13. Vorrichtung nach einem der Ansprüche 6 bis 12, **dadurch gekennzeichnet, dass** die Aufziehhülse (20) mehrteilig (30,31,32) ausgebildet ist.

14. Vorrichtung nach einem der Ansprüche 6 bis 13, **dadurch gekennzeichnet, dass** die Fluidzuführung (4) einen außenseitigen, schalt- oder steuerbaren Fluidanschluss (5) am Halter (7) und einen am Durchführkanal (15) mündenden Fluidzufluss (17) aufweist.

15. Vorrichtung nach einem der Ansprüche 6 bis 14, **dadurch gekennzeichnet, dass** die Aufziehhülse (20) eine in den Hülsenkanal (23) integrierte Dichtung (19) und am Hülsenmantel einen mit dem Fluidzufluss (17) verbindbaren Fluideinlass (35) aufweist.

16. Vorrichtung nach einem der Ansprüche 6 bis 15, **dadurch gekennzeichnet, dass** die Aufziehhülse (20) eine angeschrägte Vorderseite (25) aufweist.

17. Vorrichtung nach einem der Ansprüche 6 bis 16, **dadurch gekennzeichnet, dass** mehrere austauschbare Aufziehhülsen (20) mit unterschiedlichen Durchmessern des Hülsenkanals (23) vorgesehen sind.

18. Vorrichtung nach einem der Ansprüche 6 bis 17, **dadurch gekennzeichnet, dass** die Applikationsvorrichtung (1) mehrere austauschbare Dichtungen (19) mit unterschiedlichen Innendurchmessern aufweist.

19. Vorrichtung nach einem der Ansprüche 6 bis 18, **dadurch gekennzeichnet, dass** der Schlauch (2) aus einem transparenten, körperverträglichen Material, insbesondere aus Silicon, besteht.

20. Vorrichtung nach einem der Ansprüche 6 bis 19, **dadurch gekennzeichnet, dass** mindestens ein weiterer, über dem elastischen Schlauch (2) applizierbarer und weniger dehnbarer Schlauch oder eine Hülle (2') vorgesehen ist.

## Claims

1. Method for applying thin-walled, elastically stretchable sheaths (2) to endoscopes (3), wherein the sheath (2) is pulled with its open end area (27) onto a widening pull-on shell (20) and held securely there, and the pull-on shell (20) with the pulled-on sheath end (27) is arranged in a holder (7) with a fluid supply (6), wherein the sheath (2) is widened under pressure by an introduced fluid (26), and, with the fluid space (18) sealed, the endoscope (3) is introduced into the sheath (2), **characterized in that** the pull-on shell (20) with the pulled-on sheath end (27) is arranged in a holder (7) with a one-part housing (8) and with a lateral insertion slit (37).

2. Method according to Claim 1, **characterized in that** the endoscope (3) is introduced with circumferential play through an inner shell channel (23) of the pull-on shell (20).

3. Method according to Claim 1 or 2, **characterized in that** the fluid (26) is fed into a circumferential free space (24) between the endoscope (3) and the shell channel (23).

4. Method according to Claim 1, 2 or 3, **characterized in that** the sheath (2), in the unstretched initial state, has a substantially smaller diameter than the pull-on shell (20), wherein the funnel-shaped, substantial sheath expansion bears tightly on the front face (25) of the shell, and wherein the endoscope (3) is advanced near to or into contact with the sheath expansion, and the sheath expansion progresses with the advance of the endoscope.

5. Method according to one of the preceding claims, **characterized in that**, before the endoscope (3) is introduced, a less stretchable further sheath (2') or an envelope is applied over the elastic sheath (2).

6. Device for applying elastic sheaths (2) to endoscopes (3), wherein the application device (1) has a holder (7), with a through-channel (15) and with a fluid supply (4), and at least one pull-on shell (20) and at least one seal (19), **characterized in that** the holder (7) has a one-part housing (8) with a lateral insertion slit (37).

7. Device according to Claim 6, **characterized in that** the through-channel (15) is arranged eccentrically.

8. Device according to Claim 6 or 7, **characterized in that** the insertion slit (37) extends axially along the entire channel length of the through-channel (15), wherein the slit width is smaller than the diameter of the cylindrical through-channel (15).

9. Device according to Claim 6, 7 or 8, **characterized in that** the pull-on shell (20) is adapted in diameter to the through-channel (15) and, with the pulled-on sheath (2), is guided and held with a form fit in the through-channel (15), wherein it bears with a widened shoulder (33) on the outside wall of the housing.

10. Device according to one of Claims 6 to 9, **characterized in that** the application device (1) has a fluid source (29).

11. Device according to one of Claims 6 to 10, **characterized in that** the through-channel (15) has inlet and outlet openings (13, 14) at its ends and, between these, a receiving area (16) for the pull-on shell (20) and the sheath (2).

12. Device according to one of Claims 6 to 11, **characterized in that** the pull-on shell (20) has an inner shell channel (23) for the endoscope (3) and, on the outside, has a shoulder (33).

13. Device according to one of Claims 6 to 12, **characterized in that** the pull-on shell (20) is designed in several parts (30, 31, 32).

14. Device according to one of Claims 6 to 13, **characterized in that** the fluid supply (4) has a switchable or controllable fluid attachment (5) on the outside of the holder (7) and a fluid admission line (17) opening into the through-channel (15).

15. Device according to one of Claims 6 to 14, **characterized in that** the pull-on shell (20) has a seal (19) integrated in the shell channel (23) and, on the shell jacket, it has a fluid inlet (35) that can be connected to the fluid admission line (17).

16. Device according to one of Claims 6 to 15, **characterized in that** the pull-on shell (20) has a bevelled front face (25).

17. Device according to one of Claims 6 to 16, **characterized in that** several exchangeable pull-on shells (20) with different diameters of the shell channel (23) are provided.

18. Device according to one of Claims 6 to 17, **characterized in that** the application device (1) has several exchangeable seals (19) with different internal diameters.

19. Device according to one of Claims 6 to 18, **characterized in that** the sheath (2) is made of a transparent biocompatible material, in particular silicone.

20. Device according to one of Claims 6 to 19, **characterized in that** at least one further and less stretchable sheath or an envelope (2') is provided that can be applied over the elastic sheath (2).

## Revendications

1. Procédé pour appliquer des tuyaux flexibles (2) élastiquement extensibles et à paroi mince sur des endoscopes (3), le tuyau flexible (2) étant étiré par sa partie d'extrémité ouverte (27) sur une douille d'enfilement (20) évasée et y étant immobilisé, la douille d'enfilement (20) étant disposée avec l'extrémité (27) du tuyau flexible qui y est enfilé dans un support (7) doté d'une amenée (6) de fluide, le tuyau flexible (2) étant élargi sous pression par un fluide (26) qui y est apporté, l'endoscope (3) étant ainsi inséré dans le tuyau flexible (2) en fermant hermétiquement l'espace (18) pour le fluide, **caractérisé en ce que**
la douille d'enfilement (20) est disposée avec l'extrémité (27) du tuyau flexible qui y est enfilée dans un support (7) qui présente un boîtier (8) d'une seule pièce et une fente latérale d'insertion (37).

2. Procédé selon la revendication 1, **caractérisé en ce que** l'endoscope (3) est inséré avec un jeu périphérique par un canal (23) situé à l'intérieur de la douille d'enfilement (20).

3. Procédé selon les revendications 1 ou 2, **caractérisé en ce que** le fluide (26) est injecté dans un espace périphérique libre (24) situé entre l'endoscope (3) et le canal (23) de la douille.

4. Procédé selon les revendications 1, 2 ou 3, **caractérisé en ce que** le tuyau flexible (2) présente à l'état initial non étiré un diamètre essentiellement plus petit que la douille d'enfilement (20), l'important élargissement du tuyau flexible en forme d'entonnoir reposant hermétiquement sur le côté avant (25) de la douille et l'endoscope (3) étant avancé jusqu'à proximité de l'élargissement du tuyau flexible ou jusqu'à venir y buter, l'élargissement du tuyau flexible se poursuivant avec l'avancement de l'endoscope.

5. Procédé selon l'une des revendications précédentes, **caractérisé en ce qu'**avant l'insertion de l'endoscope (3), un autre tuyau flexible (2') moins extensible ou une enveloppe sont passés au-dessus du tuyau flexible élastique (2).

6. Dispositif d'application de tuyaux flexibles (2) sur des endoscopes (3), dans lequel le dispositif d'application (1) présente un support (7) doté d'un canal de traversée (15) et d'une amenée (4) de fluide ainsi que d'au moins une douille d'enfilement (20) et d'au moins un joint d'étanchéité (19),
**caractérisé en ce que**
le support (7) présente un boîtier (8) d'une seule pièce doté d'une fente latérale d'insertion (37).

7. Dispositif selon la revendication 6, **caractérisé en ce que** le canal de traversée (15) est disposé en position décentrée.

8. Dispositif selon les revendications 6 ou 7, **caractérisé en ce que** la fente d'insertion (37) s'étend axialement sur toute la longueur du canal de traversée (15), la largeur de la fente étant inférieure au diamètre du canal cylindrique de traversée (15).

9. Dispositif selon les revendications 6, 7 ou 8, **caractérisé en ce que** le diamètre de la douille d'enfilement (20) est adapté au canal de traversée (15) et est guidé et maintenu en correspondance géométrique dans le canal de traversée (15) avec le tuyau flexible (2) qui y est enfilé, de sorte que la douille s'appuie sur la paroi extérieure du boîtier par un collet (33) élargi.

10. Dispositif selon l'une des revendications 6 à 9, **caractérisé en ce que** le dispositif d'application (1) présente une source (29) de fluide.

11. Dispositif selon l'une des revendications 6 à 10, **caractérisé en ce que** le canal de traversée (15) présente des ouvertures frontales d'amenée et de sortie (13, 14) avec entre elles une partie de réception (16) de la douille d'enfilement (20) et du tuyau flexible (2).

12. Dispositif selon l'une des revendications 6 à 11, **caractérisé en ce que** la douille d'enfilement (20) présente un canal intérieur (23) pour l'endoscope (3) et du côté extérieur un collet (33).

13. Dispositif selon l'une des revendications 6 à 12, **caractérisé en ce que** la douille d'enfilement (20) est réalisée en plusieurs pièces (30, 31, 32).

14. Dispositif selon l'une des revendications 6 à 13, **caractérisé en ce que** l'amenée (4) de fluide présente sur le support (7) un raccordement extérieur (5) au fluide qui peut être commuté ou commandé et une amenée (17) de fluide qui débouche dans le canal de traversée (15).

15. Dispositif selon l'une des revendications 6 à 14, **caractérisé en ce que** la douille d'enfilement (20) présente un joint d'étanchéité (19) intégré dans le canal (23) de la douille et sur l'enveloppe de la douille une entrée (35) de fluide qui peut être reliée à l'amenée (17) de fluide.

16. Dispositif selon l'une des revendications 6 à 15, **caractérisé en ce que** la douille d'enfilement (20) présente un côté avant (25) chanfreiné.

17. Dispositif selon l'une des revendications 6 à 16, **caractérisé en ce que** plusieurs douilles d'enfilement remplaçables (20) présentant différents diamètres du canal (23) de la douille sont prévues.

18. Dispositif selon l'une des revendications 6 à 17, **caractérisé en ce que** le dispositif d'application (1) présente plusieurs joints d'étanchéité (19) remplaçables dont les diamètres intérieurs sont différents.

19. Dispositif selon l'une des revendications 6 à 18, **caractérisé en ce que** le tuyau flexible (2) est constitué d'un matériau transparent et compatible avec le corps, et en particulier de silicone.

20. Dispositif selon l'une des revendications 6 à 19, **caractérisé en ce qu'**il présente au moins un autre tuyau flexible qui peut être appliqué sur le tuyau flexible (2) élastique et moins extensible, ou une enveloppe (2').
